# EUROPEAN PATENT SPECIFICATION

(11) **EP 0 080 312 B1**
(45) Date of publication and mention of the grant of the patent: **29.05.1985**
(21) Application number: 82306061.1
(22) Date of filing: 15.11.1982
(51) Int. Cl.: C07C 76/02, C07C 79/46

(54) **The preparation of certain diphenyl ethers especially 5-(4-trifluoromethylphenoxy)- and 5-(2-halo-4-trifluoromethylphenoxy)-2-nitrobenzoic acid and salts and esters and amides**
Herstellung von bestimmten Diphenyläthern, insbesondere 5-(4-Trifluormethylphenoxy)- und 5-(2-Halo-4-trifluormethylphenoxy)-2-nitrobenzoesäuren und Salzen und Estern davon
Préparation de certaines éthers diphényliques, en particulier l'acide 5-(4-trifluorméthylphénoxy)-2-nitrobenzoique et l'acide 5-(2-halo-4-trifluorométhylphénoxy)-2-nitrobenzoique, leurs sels et esters

(30) Priority: 16.11.1981 US 321658
(43) Date of publication of application: 01.06.1983
(73) Proprietor: ROHM AND HAAS COMPANY, Philadelphia Pennsylvania 19105 (US)
(72) Inventor: Swithenbank, Colin, Pennsylvania 18944 (US)
(74) Representative: Wood, Peter Worsley Spencer

## Description

This invention concerns the preparation of certain diphenyl ethers especially 5-(2-halo-4-trifluoromethylphenoxy)-2-nitrobenzoic acid and salts and esters and amides.

This invention concerns more particularly a stepwise synthesis for preparing diphenyl ethers having the configuration which is currently believed to afford the most active and selective herbicide known. In this configuration the diphenyl ether has a 4-trifluoromethyl substituents, a 2-halo substituent and a 4'-nitro substituent.

The search for better and more economical methods for preparing these highly active herbicides is a continuing one. Some routes are described in U.S. Patent Nos. 3,862,209, 4,031,131 and 4,262,152. Problems also exist in the stability of trifluoromethylphenol under basic conditions [See Jones, R.G., J.A.CS., 69, 2346-50 (1947)]. The reduction of decomposition of trifluoromethylphenol and provision of a substantially pure, isomer-free compound is highly desirable.

Known processes for the preparation of these compounds yield the desired active product together with herbicidally inactive impurities especially isomers.

These inactive impurities are toxicologically disadvantageous.

EP-A-19388 and EP-A-3416 disclose the reaction of an unsubstituted p-trifluoromethylphenol with an alkyl-5-chloro-2-nitrobenzoate. However neither exemplifies such a reaction wherein the p-trifluoromethylphenol is free from any further substituent.

According to this invention there is provided a process for preparing a trifluoromethyl substituted diphenyl ether of the formula III given below wherein (A) a compound of the formula 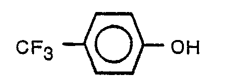 is reacted in the presence of an inorganic base in a polar solvent with a compound of the formula: 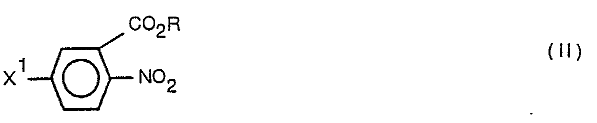 to form a compound of the formula: 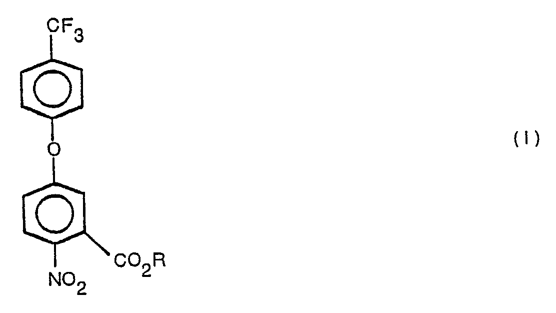 wherein X' is halo and R is hydrogen, alkyl, alkenyl, alkynyl, hydroxyalkyl, carboxyalkyl, carbalkoxyalkyl, cyanoalkyl, alkoxyalkyl, haloalkyl, alkali metal cation, alkaline earth metal cation, quarternary ammonium cation, amino, or mono-R amino or di-R amino, and then (B) the compound (I) is reacted with a halogenating agent in the presence of a Friedel-Crafts catalyst comprising ferric chloride and/or aluminium chloride to form a compound of the formula: 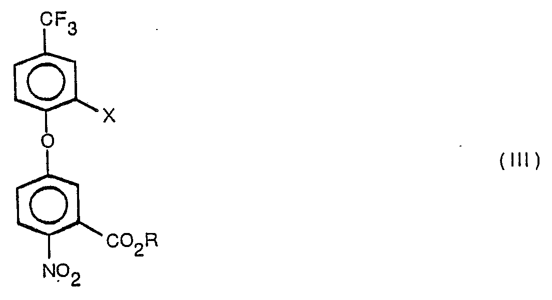 wherein X is halo and R is as defined above.

An important feature of the reactions of this invention is the formation and use of compound (I) wherein the trifluoromethylphenoxy moiety is free of halogen which is subsequently added.

A flow diagram for the process according to this invention is as follows: 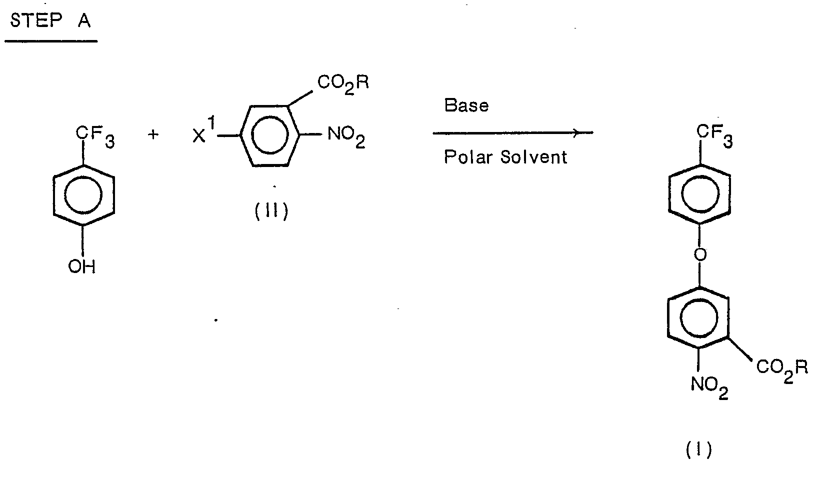 Step A is preferably carried out at a temperature of 25 to 90°C and for 1 to 18 hours. 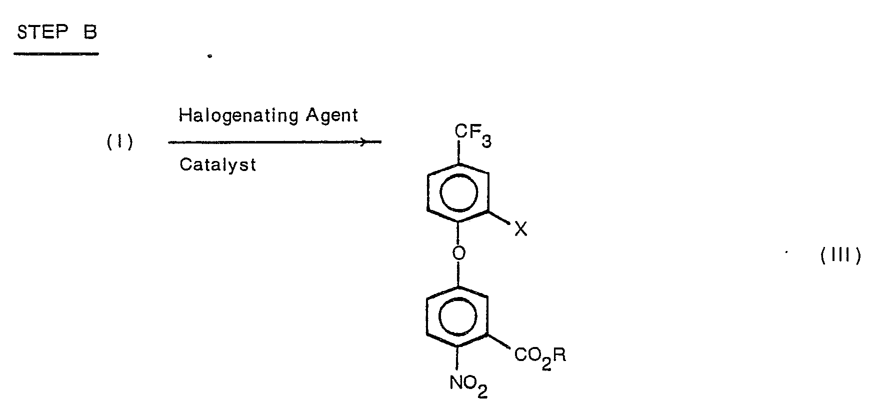 Step B is carried out in the presence of a Friedel Crafts catalyst and preferably at a temperature of 50° to 130°C and for 0.5 to 10 hours. 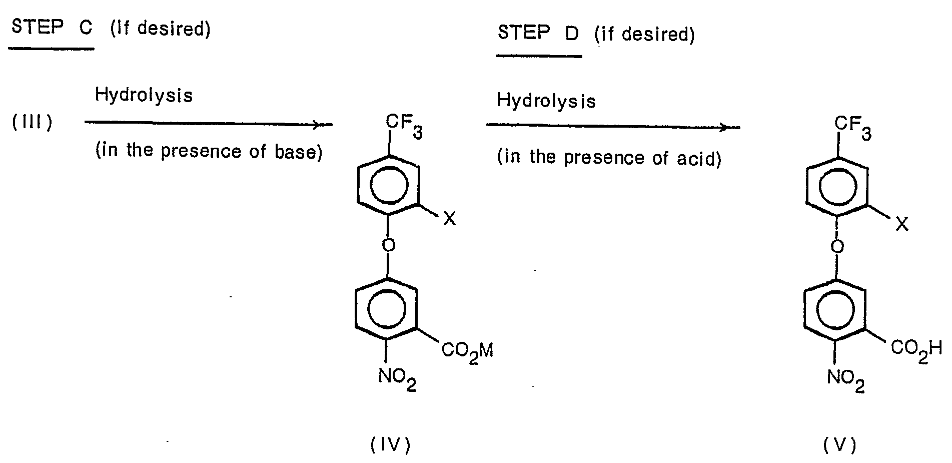 wherein M is an alkali metal cation or alkaline earth metal cation.

The condensation step, Step A, may employ inorganic bases such as sodium carbonate, potassium carbonate, sodium hydride, sodium hydroxide, potassium hydroxide, lithium hydroxide, Grignard reagents, lithium alkyls or any other base which will form the free anion of the phenol. The preferred base is potassium carbonate. This reaction is conducted using a polar solvent, such as, dimethyl sulfoxide, sulfolane, dimethylformamide, hexamethyl phosphoramide, tetramethylurea, tetramethylethylene diamine and the like. Dimethyl sulfoxide is the preferred solvent. The reaction may be conducted at a temperature in the range of from 25° to 90°C with the range of from 50° to 80°C being more preferred and 70°C being most preferred. The reaction may be carried out for a period of time within 1 to 18 hours, and within 2 to 3 hours being most preferred for completing the reaction.

Gas-liquid chromatography shows that this process can be operated so that only the desired product is obtained and yields of over 80% may be obtainable.

The halogenation step, Step B, can be so operated as to yield, out of five possible isomers, only the single desired product (mono-halogenated compound III with halogen at the 2-position). While only chlorinating agents have been employed, it is believed that other halogenating agents could be equally effective such as fluorinating or iodinating agents.

Halogenating agents which can be employed include gaseous chlorine, sulfuryl chloride, N-chlorosuccinimide, tert--butyl hypochlorite, hydrogen chloride plus oxidizing agents (e.g. perchlorate) with gaseous chlorine being the preferred chlorinating agent.

The Friedel-Crafts catalyst employed in Step 8 comprises ferric chloride and/or aluminium chloride. The reaction time may be quite short, from 0.5 to 10 hours, at a temperature from 5°C to 130°C. The reaction is preferably run for no more than 3 hours at 70°C. A solvent may optionally be employed although, in general, none is needed. Solvents which could be used include chlorinated solvents such as o-dichlorobenzene, perchloroethylene or ethylene dichloride.

The product (compound III) may be employed directly as a herbicide or may be converted to other herbicides by hydrolysis of the ester, salt or amide to the free acid which can be converted to other esters, alkali metal or alkaline earth metal salts or to various amine salts as desired.

The following is a description of the various substituents, X, X', and R. X and X' are halo, for example, chloro, bromo, fluoro, or iodo.

R may be one of the following: hydrogen, alkyl, such as lower alkyl of from 1 to 5 carbon atoms, alkenyl, such as lower alkenyl of from 2 to 6 carbon atoms, alkynyl, such as lower alkynyl of from 2 to 6 carbon atoms, hydroxyalkyl such as hydroxy (C, to Cₛ)-alkyl, carboxyalkyl such as carboxy (C, to Cs)-alkyl, carbalkoxyalkyl, such as carbo (C, to C₅)-alkoxy (C₁ to C₅)-alkyl, cyanoalkyl such as, cyano (C₁ to C₅)-alkyl, alkoxyalkyl, such as (C₁ to C₅)-alkoxy (C₁ to C₅)-alkyl, haloalkyl such as halo (C₁ to C₅)-alkyl, a cation derived from an alkali metal or an alkaline earth metal such as Na⁺_{'} K⁺, Li⁺, Ca⁺⁺, Mg⁺⁺, quaternary ammonium cations or amino, mono- and di-R amino where R is as defined above. [Note: 1) When R is hydrogen, two equivalents of base must be employed; 2) R cannot be hydroxylmethyl; and 3) for the halogenation, R is preferably (C₁ to Cₛ)-alkyl.]

Examples of processes according to the invention are given below for illustrative purposes only.

### Example 1

### 5-(2-Chloro-4-trifluoromethylphenoxy)-2-nitrobenzoic acid and its Sodium Salt

### Step A - Methyl 5-(4-trifluoromethylphenoxy)-2-nitro-benzoate

4-trifluoromethylphenol (0.22 g, 1.36 mmol), powdered potassium carbonate (0.185 g, 1.34 mmol), and methyl 5-chloro-2-nitrobenzoate (0.288 g, 1.34 mmol) were heated at 70°C under a nitrogen atmosphere in 3 ml of dimethyl sulfoxide. After 50 minutes, an aliquot was quenched in methanolic HCI. (By gas chromatography, the reaction was 45% complete). After 18 hours (overnight), the reaction mixture was cooled to room temperature, diluted with water, and extracted with ether. The organic extracts were shaken with brine, dried (MgS0₄) and concentrated to afford methyl 5-(4-trifluoromethylphenoxy)-2-nitrobenzoate in 85% yield. Gas chromatography showed no starting phenol remained.

### Step B - Methyl 5-(2-chloro-4-trifluoromethylphenoxyJ-2-nitrobenzoate

Methyl 5-(4-trifluoromethylphenoxy)-2-nitrobenzoate was stirred with a catalytic amount of ferric chloride. The reaction mixture was heated to 70°C under a continuous stream of chlorine gas. After 2.5 hours, the mixture was taken up in ether, shaken with water and the organic phase was dried over MgS0₄. The ether was evaporated to afford methyl 5-(2-chloro-4-trifluoromethylphenoxy)-2-nitrobenzoate. NMR (CDCI₃) 6 8.2-7 (m, 6) 4.0 (2, 3). TLC (40% ether/hexane) R_{f} = 4. GL was unable to detect the presence of any impurity. The yield of desired product was quantitative.

### Step C - 5-(2-Chloro-4-trifluoromethylphenoxy)-2-nitrobenzoic acid and its Sodium Salt

Methyl 5-(2-chloro-4-trifluoromethylphenoxy)-2-nitrobenzoate (0.06 mole) was dissolved in methanol (20 g) and a solution of 50% aqueous sodium hydroxide (0.120 mole) was added dropwise beginning at 25°C. The rate of addition was adjusted so as to control the exothermic reaction at less than or equal to 52°C. By this method the sodium salt of 5-(2-chloro-4-trifluoromethylphenoxy)-2-nitrobenzoic acid was generated in situ. Treatment with 6 M HCI to pH 1 in water (100 ml) afforded the free acid which was extracted into ethylene chloride. The ethylene chloride layer was decanted and concentrated under vacuum to afford 5-(2-chloro-4-trifluoromethylphenoxy)-2-nitrobenzoic acid.

By standard chemical methods 5-(2-chloro-4-trifluoromethylphenoxy)-2-nitrobenzoic acid can be converted to agronomically acceptable alkali and alkaline earth metal salts by reaction with one equivalent of an appropriate metal hydroxide or to agronomically acceptable amine salts by combining the acid with one equivalent of the appropriate amine. Solutions of the acid salts orfree salts can be obtained depending upon reaction conditions. One method for illustrative purposes only to isolate the sodium salt is described below.

### Example 1A

### Sodium 5-(2-Chloro-4-trifluoromethylphenoxy)-2-nitrobenzoate

5-(2-Chloro-4-trifluoromethylphenoxy)-2-nitrobenzoic acid (3.0 g, 0.0083 mole) was dissolved in isopropyl alcohol (20 ml) and a solution of 50% aqueous sodium hydroxide (0.644 g, 0.0083 mole) was added dropwise and the reaction mixture stirred for 1 hour before removing the solvent and water under vacuum at 0.2 mm overnight to afford 2.8 g of sodium-(2-chloro-4-trifluoromethylphenoxy)-2-nitrobenzoate.

By following substantially the same procedure as described above the following compounds were prepared:

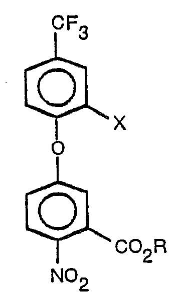

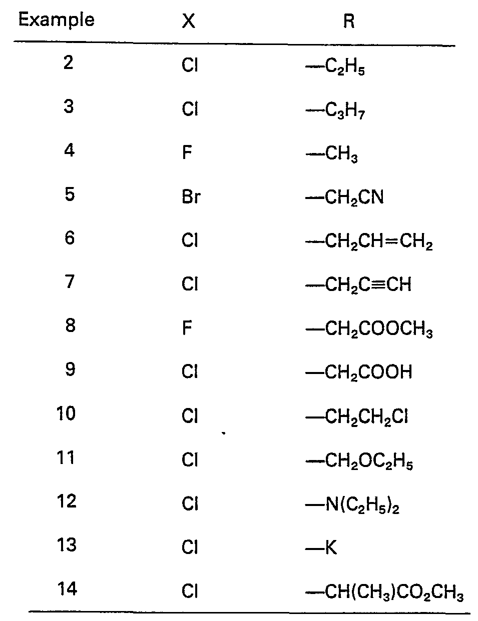

## Claims

1. A process for preparing a trifluoromethyl substituted diphenyl ether of the formula III given below characterised in that (A) a compound of the formula 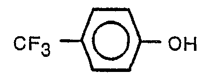 is reacted in the presence of an inorganic base in a polar solvent with a compound of the formula: 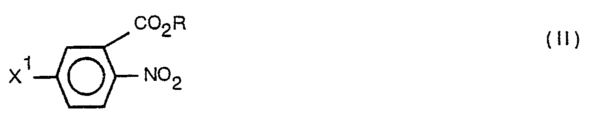 to form a compound of the formula: 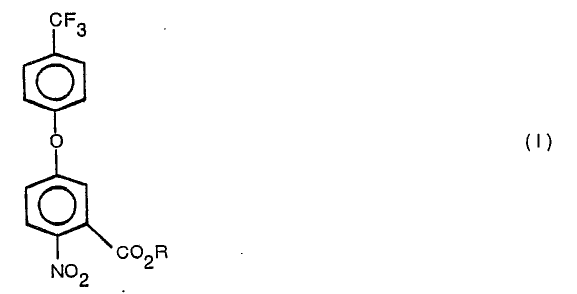 wherein X' is halo and R is hydrogen, alkyl, alkenyl, alkynyl, hydroxyalkyl, carboxyalkyl, carbalkoxyalkyl, cyanoalkyl, alkoxyalkyl, haloalkyl, alkali metal cation, alkaline earth metal cation, quarternary ammonium cation, amino, or mono-R amino or di-R amino, and then (B) this compound (I) is reacted with a halogenating agent in the presence of a Friedel-Crafts catalyst comprising ferric chloride and/or aluminium chloride to form a compound of the formula: 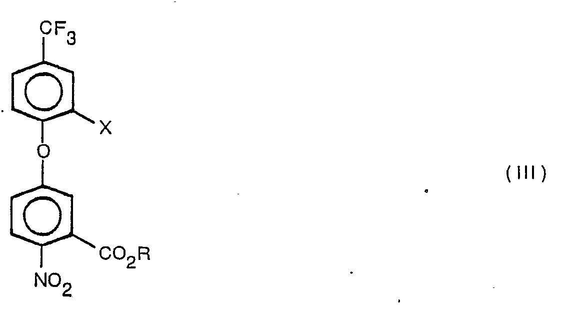 wherein X is halo and R is as defined above.

2. A process as claimed in Claim 1 wherein reaction (A) is carried out at 25°C to 90°C for 1 to 18 hours.

3. A process as claimed in Claim 1 or 2 wherein reaction (B) is carried out at 50°C to 130°C for 0.5 to 10 hours.

4. A process as claimed in any preceding claim wherein compound (III) is hydrolyzed in the presence of base to form: 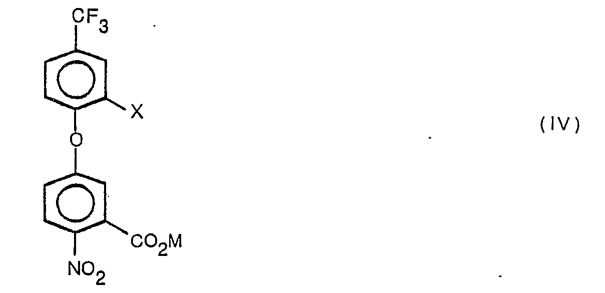 wherein M is an alkali metal cation or alkaline earth metal cation and X is halo.

5. A process as claimed in Claim 5 wherein compound (IV) is hydrolysed in the presence of acid to form: 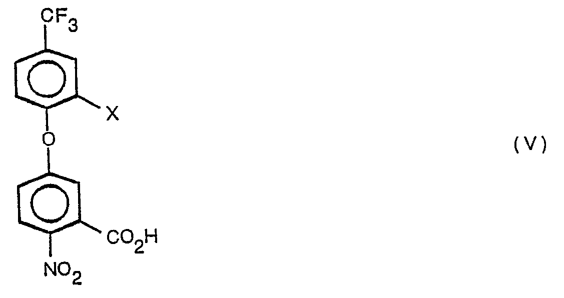 wherein X is halo.

6. A process as claimed in Claim 4 or 5 wherein X is CI and M is Na.

7. A process as claimed in any of Claims 1 to 5 which comprises reacting 4-trifluoromethylphenol with methyl 5-chloro-2-nitrobenzoate and potassium carbonate in dimethyl sulfoxide followed by treating the compound (I) obtained with chlorine gas in the presence of ferric chloride to form an ester (III), then hydrolysing the ester (lll) with aqueous base to form sodium 5-(2-chloro-4-trifluoromethylphenoxy)-2-nitrobenzoate.

8. A process as claimed in Claim 7 wherein the sodium 5-(2-chloro-4-trifluoromethylphenoxy)-2-nitrobenzoate is reacted with inorganic acid to form 5-(2-chloro-4-trifluoromethylphenoxy)-2-nitrobenzoic acid.

## Patentansprüche

1. Verfahren zur Herstellung eines Trifluormethylsubstituierten Diphenylethers der nachfolgenden Formel 111, dadurch gekennzeichnet, daß (A) eine Verbindung der Formel 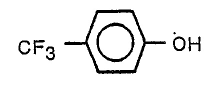 in Gegenwart einer anorganischen Base in einem polaren Lösungsmittel mit einer Verbindung der Formel 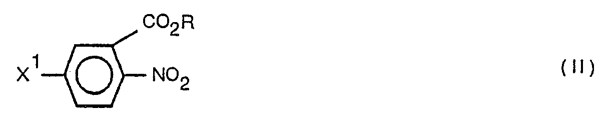 unter Bildung einer Verbindung der Formel 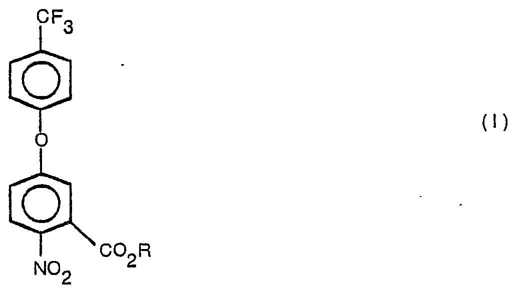 worin X' für Halogen steht und R Wasserstoff, Alkyl, Alkenyl, Alkinyl, Hydroxyalkyl, Carboxyalkyl, Carbalkoxyalkyl, Cyanoalkyl, Alkoxyalkyl, Halogenalkyl, ein Alkalimetallkation, ein Erdalkalimetallkation, ein quaternäres Ammoniumkation, Amino oder mono-R-Amino oder di-R-Amino bedeutet, umgesetzt wird, und dann (B) diese Verbindung (I) mit einem Halogenierungsmittel in Gegenwart eines Friedel-Crafts-Katalysators aus Eisen (III) chlorid und/oder Aluminiumchlorid unter Bildung einer Verbindung der Formel 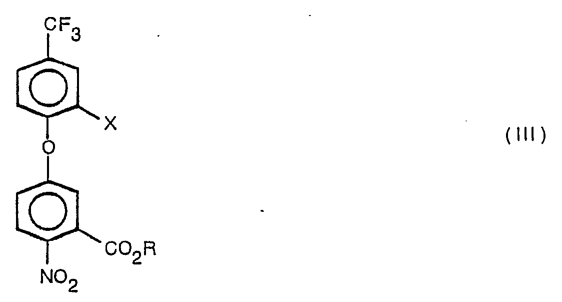 zur Umsetzung gebracht wird, worin X für Halogen steht und R die vorstehend angegebene Bedeutung besitzt.

2. Verfarhen nach Anspruch 1, dadurch gekennzeichnet, daß die Reaktion (A) bei 25 bis 90°C während 1 bis 18 Stunden durchgeführt wird.

3. Verfahren nach Anspruch 1 oder 2, dadurch gekennzeichnet, daß die Reaktion (B) bei 50 bis 130°C während 0,5 bis 10 Stunden durchgeführt wird.

4. Verfahren nach einem der vorhergehenden Ansprüche, dadurch gekennzeichnet, daß die Verbindung (111) in Gegenwart einer Base unter Bildung 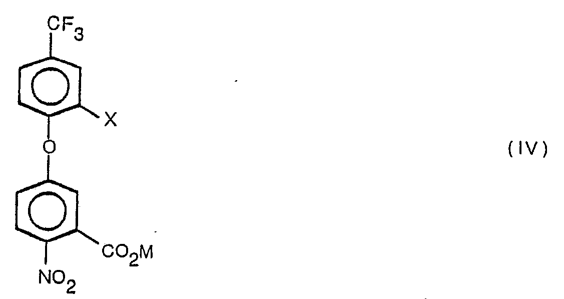 hydrolysiert wird, worin M ein Alkalimetallkation oder ein Erdalkalimetallkation ist und X für Halogen steht.

5. Verfahren nach Anspruch 4, dadurch gekennzeichnet, daß die Verbindung (IV) in Gegenwart einer Säure unter Bildung 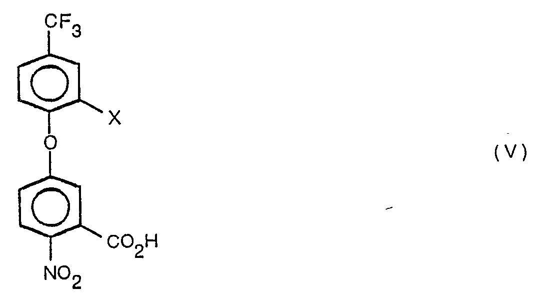 hydrolysiert wird, worin X für Halogen steht.

6. Verfahren nach Anspruch 4 oder 5, dadurch gekennzeichnet, daß X für CI steht und M Na ist.

7. Verfahren nach einem der Ansprüche 1 bis 5, dadurch gekennzeichnet, daß 4-Trifluormethylphenol mit Methyl-5-chlor-2-nitrobenzoat und Kaliumcarbonat in Dimethylsulfoxid umgesetzt wird und anschließend die erhaltene Verbindung (I) mit Chlorgas in Gegenwart von Eisen (III) chlorid unter Bildung eines Esters (III) behandelt wird und dann der Ester (III) mit einer wäßrigen Base unter Bildung von Natrium-5-(2-chlor-4-trifluormethylphenoxy)₋2-nitrobenzoat hydrolysiert wird.

8. Verfahren nach Anspruch 7, dadurch gekennzeichnet, daß das Natrium-5-(2-chlor-4-trifluormethyl- phenoxy)-2-nitrobenzoat mit einer anorganischen Säure unter Bildung von 5-(2-chlor-4-trifluormethyl- phenoxy)-2-nitrobenzoesäure umgesetzt wird.

## Revendications

1. Procédé pour préparer un éther diphénylique à substitution trifluorométhylique répondant à la formule III ci-dessous caractérisé en ce que (A) on fait réagir un composé de formule 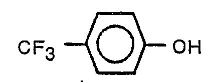 en présence d'une base minérale dans un solvant polaire avec un composé de formule: 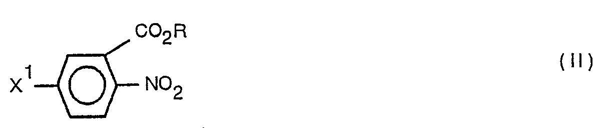 pour former un composé de formule 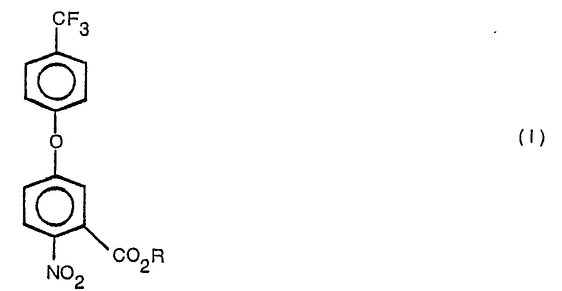 dans laquelle X' est un halogéno et R est un hydrogène, un alcoyle, un alcényle, un alcynyle, un hydroxy- alcoyle, un carboxyalcoyle, un carbalcoxyalcoyle, un cyanoalcoyle, un alcoxyalcoyle, un halogénoalcoyle, un cation de métal alcalin, un cation de métal alcalino-terreux, un cation ammonium quaternaire, un amino ou un mono-R-amino ou di-R-amino; puis (B) on fait réagir ce composé (I) avec un agent d'halogénation en présence d'un catalyseur de Friedel-Crafts comprenant du chlorure ferrique et/ou du chlorure d'aluminium pour former un composé de formule: 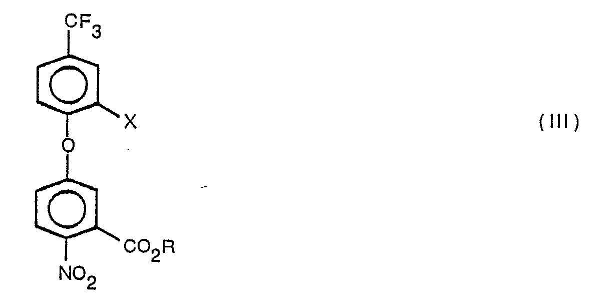 dans laquelle X est un halogéno et R est comme défini ci-dessus.

2. Procédé comme revendiqué dans la revendication 1 dans lequel la réaction (A) est effectuée entre 25° et 90°C pendant 1 à 18 heures.

3. Procédé comme revendiqué dans la revendication 1 ou 2, dans lequel la réaction (B) est effectuée entre 50°C et 130°C pendant 0,5 à 10 heures.

4. Procédé comme revendiqué dans l'une quelconque des revendications précédentes, dans lequel le composé (III) est hydrolysé en présence d'une base pour former: 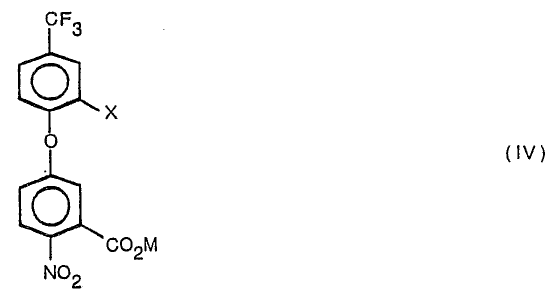 où M est un cation de métal alcalin ou un cation de métal alcalino-terreux et X est un halogéno.

5. Procédé comme revendiqué dans la revendication 5 dans lequel le composé (IV) est hydrolysé en présence d'acide pour former: 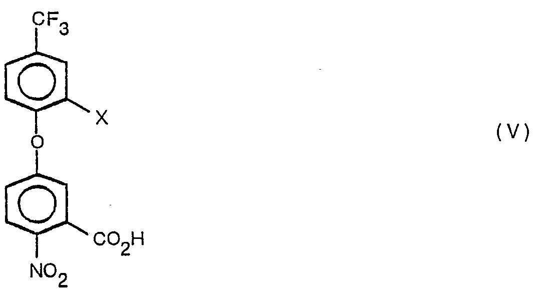 où X est un halogéno.

6. Procédé comme revendiqué dans la revendication 4 ou 5 où X est CI et M est Na.

7. Procédé comme revendiqué dans l'une quelconque des revendications 1 à 5 qui comprend la réaction du 4-trifluorométhylphénol avec le 5-chloro-2-nitrobenzoate de méthyle et le carbonate de potassium dans le diméthylsulfoxyde puis le traitement du composé (I) obtenu avec du chlore gazeux en présence de chlorure ferrique pour former un ester (III) puis l'hydrolyse de l'ester (III) avec une base aqueuse pour former le 5-(2-chloro-4-trifluorométhylphénoxy)-2-nitrobenzoate de sodium.

8. Procédé comme revendiqué dans la revendication 7 dans lequel on fait réagir le 5-(2-chloro-4-trifluorométhylphénoxy)-2-nitrobenzoate de sodium avec un acide minéral pour former l'acide 5-(2-chloro-4-trifluorométhylphénoxy)-2-nitrobenzoïque.
